# EUROPEAN PATENT SPECIFICATION

(11) **EP 2 480 575 B1**
(45) Date of publication and mention of the grant of the patent: **26.12.2018**
(21) Application number: 10766312.2
(22) Date of filing: 22.09.2010
(51) Int. Cl.: C07K 16/28, A61K 38/17, C07K 14/705, G01N 33/50, G01N 33/68, A61P 35/00

(54) **ANTAGONISTS OF DSG2 FOR TREATMENT OF CANCER**
DSG2-ANTAGONISTEN ZUR BEHANDLUNG VON KREBS
ANTAGONISTES DE DSG2 POUR LE TRAITEMENT DU CANCER

(30) Priority: 23.09.2009 GB 0916686
(43) Date of publication of application: 01.08.2012
(73) Proprietor: Asclepiumm Limited, Manchester M4 7AJ (GB)
(72) Inventor: CHEN, Min-Che, Greater Manchester M4 7AJ (GB)
(74) Representative: Stafford, Jonathan Alan Lewis
(86) International application number: PCT/GB2010/001776
(87) International publication number: WO 2011/036440

(56) References cited:
- WO-A2-99/57149
- WO-A2-2007/047796
- US-A1- 2005 203 025
- MORRIS ET AL: "A Phase II Trial of Bortezomib and Prednisone for Castration Resistant Metastatic Prostate Cancer", JOURNAL OF UROLOGY, LIPPINCOTT WILLIAMS & WILKINS, BALTIMORE, MD, US, vol. 178, no. 6, 22 October 2007 (2007-10-22), pages 2378-2384, XP022338332, ISSN: 0022-5347, DOI: DOI:10.1016/J.JURO.2007.08.015
- CODONY-SERVAT J ET AL: "Differential cellular and molecular effects of bortezomib, a proteasome inhibitor, in human breast cancer cells", MOLECULAR CANCER THERAPEUTICS, AMERICAN ASSOCIATION OF CANCER RESEARCH, US, vol. 5, no. 3, 1 March 2006 (2006-03-01), pages 665-675, XP002599501, ISSN: 1535-7163, DOI: DOI:10.1158/1535-7163.MCT-05-0147
- GREGORY B LESINSKI ET AL: "Bortezomib pre-treatment prolongs interferon-alpha-induced STAT1 phosphorylation in melanoma cells", CANCER IMMUNOLOGY, IMMUNOTHERAPY, SPRINGER, BERLIN, DE, vol. 58, no. 12, 26 April 2009 (2009-04-26), pages 2031-2037, XP019757683, ISSN: 1432-0851, DOI: DOI:10.1007/S00262-009-0710-Y
- LORCH JOCHEN H ET AL: "Bortezomib upregulates the EGF-receptor and inhibits cell-cell adhesion and cell migration in squamous cell cancer of the head and neck.", PROCEEDINGS OF THE AMERICAN ASSOCIATION FOR CANCER RESEARCH ANNUAL MEETING, vol. 48, April 2007 (2007-04), pages 951-952, XP008130750, & 98TH ANNUAL MEETING OF THE AMERICAN-ASSOCIATION-FOR-CANCER-RESEARCH; LOS ANGELES, CA, USA; APRIL 14 -18, 2007 ISSN: 0197-016X
- DAVIES ELERI LLOYD ET AL: "The role of desmoglein 2 and E-cadherin in the invasion and motility of human breast cancer cells", INTERNATIONAL JOURNAL OF ONCOLOGY, SPANDIDOS PUBLICATIONS, GR, vol. 11, no. 2, 1 January 1997 (1997-01-01), pages 415-419, XP009105589, ISSN: 1019-6439
- LU MING ET AL: "Targeted inhibition of EG-1 blocks breast tumor growth.", CANCER BIOLOGY & THERAPY JUN 2007 LNKD- PUBMED:17568184, vol. 6, no. 6, June 2007 (2007-06), pages 936-941, XP002614851, ISSN: 1555-8576
- DATABASE UniProt [Online] 25 November 2002 (2002-11-25), "O55111 (DSG2_MOUSE)", XP002614852, Database accession no. O55111
- DATABASE UniProt [Online] 1 November 1997 (1997-11-01), "Q14126 (DSG2_HUMAN)", XP002614853, Database accession no. Q14126
- KURREY N K ET AL: "Snail and Slug are major determinants of ovarian cancer invasiveness at the transcription level", GYNECOLOGIC ONCOLOGY, ACADEMIC PRESS, LONDON, GB, vol. 97, no. 1, 1 April 2005 (2005-04-01), pages 155-165, XP004801778, ISSN: 0090-8258, DOI: DOI:10.1016/J.YGYNO.2004.12.043
- SCHAFER S ET AL: "Immunological identification and characterization of the desmosomal cadherin Dsg2 in coupled and uncoupled epithelial cells and in human tissues", DIFFERENTIATION, SPRINGER VERLAG, DE, vol. 60, no. 2, 1 May 1996 (1996-05-01), pages 99-108, XP026792148, ISSN: 0301-4681 [retrieved on 1996-05-01]
- KARTENBECK J ET AL: "Synthesis of junctional proteins in metastasizing colon cancer cells", EUROPEAN JOURNAL OF CELL BIOLOGY, WISSENSCHAFLICHE VERLAGSGESELLSCHAFT, STUTTGART, DE, vol. 84, no. 2-3, 9 March 2005 (2005-03-09), pages 417-430, XP004954687, ISSN: 0171-9335, DOI: 10.1016/J.EJCB.2005.01.005
- JIANG XIAN PENG ET AL: "Vaccination with a mixed vaccine of autogenous and allogeneic breast cancer cells and tumor associated antigens CA15-3, CEA and CA125: Results in immune and clinical responses in breast cancer patients", 20000101; 20000000, vol. 15, no. 5, 1 January 2000 (2000-01-01), pages 495-505, XP002374854,
- PRÉVOST G ET AL: "Molecular heterogeneity of somatostatin analogue BIM-23014C receptors in human breast carcinoma cells using the chemical cross-linking assay.", CANCER RESEARCH 15 FEB 1992, vol. 52, no. 4, 15 February 1992 (1992-02-15), pages 843-850, ISSN: 0008-5472
- MOLL R ET AL: "Molekulare Diversitat der desmosomalen Cadherine und ihr Potential als Marker in der Histodiagnose von Karzinomen - [Molecular diversity of desmosomal cadherins and their potential as markers in the histodiagnosis of carcinomas]", VERHANDLUNGEN DER DEUTSCHEN GESELLSCHAFT FUER PATHOLOGIE, GUSTAV FISCHER VERLAG, STUTTGART, DE, vol. 78, 1 January 1994 (1994-01-01), pages 277-284, XP008180753, ISSN: 0070-4113

## Description

The present invention relates to agents for use in treating cancer.

Cancer is a class of diseases in which a group of cells display uncontrolled growth (division beyond the normal limits), invasion (intrusion on and destruction of adjacent tissues), and sometimes metastasis (spread to other locations in the body via lymph or blood). These three malignant properties of cancers differentiate them from benign tumors, which are self-limited, and do not invade or metastasize. Most cancers form a tumor but some, like leukemia, do not.

Cancer is caused by both external factors (tobacco, infectious organisms, chemicals, and radiation) and internal factors (inherited mutations, hormones, immune conditions, and mutations that occur from metabolism). These causal factors may act together or in sequence to initiate or promote carcinogenesis. Cancer may affect people at all ages, even fetuses, but the risk for most varieties increases with age. Cancer causes about 13% of all human deaths. According to the American Cancer Society, 7.6 million people died from cancer in the world during 2007. Diagnosis usually requires the histologic examination of a tissue biopsy specimen by a pathologist, although the initial indication of malignancy can be symptoms or radiographic imaging abnormalities.

Nearly all cancers are caused by abnormalities in the genetic material of the transformed cells. These abnormalities may be due to the effects of carcinogens, such as tobacco smoke, radiation, chemicals, or infectious agents. Other cancer-promoting genetic abnormalities may be randomly acquired through errors in DNA replication, or are inherited, and thus present in all cells from birth. The heritability of cancers is usually affected by complex interactions between carcinogens and the host's genome. New aspects of the genetics of cancer pathogenesis, such as DNA methylation, and microRNAs are increasingly recognized as important.

Genetic abnormalities found in cancer typically affect two general classes of genes. Cancer-promoting oncogenes are typically activated in cancer cells, giving those cells new properties, such as hyperactive growth and division, protection against programmed cell death, loss of respect for normal tissue boundaries, and the ability to become established in diverse tissue environments. Tumor suppressor genes are then inactivated in cancer cells, resulting in the loss of normal functions in those cells, such as accurate DNA replication, control over the cell cycle, orientation and adhesion within tissues, and interaction with protective cells of the immune system.

Most cancers can be treated and some cured, depending on the specific type, location, and stage. Once diagnosed, cancer is usually treated with a combination of surgery, chemotherapy and radiotherapy. As research develops, treatments are becoming more specific for different varieties of cancer. There has been significant progress in the development of targeted therapy drugs that act specifically on detectable molecular abnormalities in certain tumors, and which minimize damage to normal cells. The prognosis of cancer patients is most influenced by the type of cancer, as well as the stage, or extent of the disease. In addition, histologic grading and the presence of specific molecular markers can also be useful in establishing prognosis, as well as in determining individual treatments.

Metastasis is the spread of a disease, such as cancer, from one organ or part to another non-adjacent organ or part. Cancer cells can break away, leak, or spill from a primary tumor, enter lymphatic and blood vessels, circulate through the bloodstream, and settle down to grow within normal tissues elsewhere in the body. Metastasis is one of three hallmarks of malignancy (contrast benign tumors). Most tumors and other neoplasms can metastasize, although in varying degrees (e.g., glioma and basal cell carcinoma rarely metastasize). When tumor cells metastasize, the new tumor is called a secondary or metastatic tumor, and its cells are like those in the original tumor. This means, for example, that, if breast cancer metastasizes to the lungs, the secondary tumor is made up of abnormal breast cells, not of abnormal lung cells. The tumor in the lung is then called metastatic breast cancer, not lung cancer.

Treatment and survival is determined by whether or not a cancer is local or has spread to other locations. If the cancer spreads to other tissues and organs, it may decrease a patient's likelihood of survival. However, there are some cancers (i.e., leukemia, cancer of the brain) that can kill without spreading at all. When cancer has metastasized, it may be treated with radiosurgery, chemotherapy, radiation therapy, biological therapy, hormone therapy, surgery or a combination of these. The choice of treatment generally depends on the type of primary cancer, the size and location of the metastasis, the patient's age and general health, and the types of treatments used previously. In patients diagnosed with CUP, it is still possible to treat the disease even when the primary tumor cannot be located. The treatment options currently available are rarely able to cure metastatic cancer.

Davies Eleri Lloyd et al: "The role of desmoglein 2 and E-cadherin in the invasion and motility of human breast cancer cells", International Journal of Oncology, Spandidos Publications, GR, vol. 11, no. 2, 1 January 1997 (1997-01-01), pages 415-419, discloses that MCF7 cells express Dsg2 and that statistically significant (page 418, right column, lines 24-25). Davies *et al* come to the conclusion that inhibiting Dsg2 increases motility and invasion of breast cancer cells.

WO 99/57149 discloses compounds and methods for modulating nonclassical cadherin-mediated functions and discloses a 113 amino acid peptide which consists of the amino acid sequence TQDVFVGSVEELSAAHTLVMKINATDADEPNTLNSKISYR IVSLEPAYPP VFYLNKDTGE IYTTSVTLDR EEHSSYTLTV EARDGNGEVT DKPVKQAQVQ IRILDVNDNI PVV

US 2005/203025 suggests that CAR-regions are involved in mediating Dsg2-function. Specifically the sequences VFYLNKDTGE (SEQ. 3290) and LTGYALDARG (SEQ. 4100) are mentioned.

Against this background, it is clearly desirable to identify new agents that can be used for the treatment of cancer.

Desmosomes are one of the principal types of cell-cell adhesion junction between epithelial, myocardial and other tissues. Such desmosomes contain transmembrane glycoproteins called desmosomal cadherin, desmocollin (Dsc) and desmoglein (Dsg). Each occurs as at least three distinct genetic isoforms that show tissue-specific expression patterns.

Dsg2 are ubiquitously expressed in all tissues that form desmosomes. The extracellular domains of Dsg2 contain four cadherin repeat domains (EC1-4), each about 110 amino acids each in length. The extracellular repeat domain EC1 contains cell adhesion recognition (CAR) sites, which provide cell-cell adhesion. Therefore, Dsg2 has been identified to be a transmembrane cell adhesion molecule. Additionally, recent studies show that Dsg2 is not just a simple cell-cell adhesion molecule. Dsg2 is involved in promotion of angiogenesis, signalling of apoptosis, and is a substrate for MMPs.

The inventors have now determined that Dsg2 has an important role in regulating epithelial-mesenchymal transition (EMT) in cells. EMT is a program of development of cells characterized by loss of cell adhesion and increased cell mobility. EMT is essential for numerous developmental processes including mesoderm formation and neural tube formation. EMT also plays a central role during tumour progression, invasion and metastasis. Cancer cells undergo EMT to lose cell adhesion and acquire mesenchymal characteristics, some of which are necessary for invasion and metastasis.

Davies et al (International Journal of Oncology, vol.11, no.2, 1 January 1997, 415-419) describes the expression of Dsg2 in breast cancer cells and assesses the role of Dsg2 in invasion and motility. Davies showed that 3 breast cancer cell lines express Dsg2 and cell aggregation was reduced, *in vitro* invasion and motility were increased in Dsg2 Mab pre-treated cells. The findings indicate that Dsg2 present in breast cancer cells may act as a tumour suppressor molecule.

The invention relates to antagonists of Dsg2 for use as a medicament for preventing or treating cancer, wherein said antagonists modulate the function of the amino acid sequence: TQDVFVGSVEELSAAHTLVMKINATDADEPNTLNSKISYR (SEQ ID NO:1), or a fragment or variant thereof, of the EC2 domain of Dsg2.

A first aspect of the invention provides a monoclonal antibody as an antagonist for use as a medicament for preventing or treating cancer, characterised in that the monoclonal antibody specifically binds to the amino acid sequence: TQDVFVGSVEELSAAHTLVMKINATDADEPNTLNSKISYR (SEQ ID NO:1), KINATDADEPNTLNSKISYR (SEQ ID NO:6) or EELSAAHTLV (SEQ ID NO:7) of the non-cell adhesion recognition (CAR) sites region of the EC2 domain of Dsg2 and inhibits epithelial mesenchymal transition (EMT).

A second aspect of the invention provides a polyclonal antibody as an antagonist for use as a medicament for preventing or treating cancer, characterised in that the polyclonal antibody is raised against the amino acid sequence TQDVFVGSVEELSAAHTLVMKINATDADEPNTLNSKISYR (SEQ ID NO:1) or SEQ ID NO:5 and inhibits epithelial mesenchymal transition (EMT).

A third aspect of the invention provides a polypeptide comprising
(a) the amino acid sequence TQDVFVGSVEELSAAHTLVMKINATDADEPNTLNSKISYR (SEQ ID NO:1), KINATDADEPNTLNSKISYR (SEQ ID NO:6) or EELSAAHTLV (SEQ ID NO:7) wherein said amino acid sequence is capable of inhibiting the epithelial mesenchymal transition (EMT) promoting function of Dsg2; or
(b) a biologically active fragment of the amino acid sequence TQDVFVGSVEELSAAHTLVMKINATDADEPNTLNSKISYR (SEQ ID NO:1), KINATDADEPNTLNSKISYR (SEQ ID NO:6) or EELSAAHTLV (SEQ ID NO:7) wherein said fragment thereof is capable of inhibiting the epithelial mesenchymal transition (EMT) promoting function of Dsg2
for use as a medicament for preventing or treating cancer.

A fourth aspect of the invention provides an isolated polypeptide comprising
(a) the amino acid sequence SEQ ID NO:9 wherein said amino acid sequence is capable of inhibiting the epithelial mesenchymal transition (EMT) promoting function of Dsg2
for use as a medicament for preventing or treating cancer.

As mentioned above, the inventors have determined that Dsg2 has an important role in regulating EMT. They have shown that: (1) Triggering EMT using hepatocyte growth factor/scattering factor (HGF/SF) shows that most of the desmosomal adhesion components are down-regulated, except Dsg2. (2) Epithelial cells transfected with Dsg2 exhibit a mesenchymal-like morphology and showed greater migration and invasion abilities under treatment by HGF/SF. (3) Antibodies against EC2 domain of Dsg2 significantly block HGF/SF-induced EMT *in vitro.* Furthermore, the inventors have determined that antibodies to the EC2 domain of Dsg2 inhibit invasion of cancer cells, including MCF7 human breast cancer cells, LNCaP human prostate cancer cells, and KM12 human colon cancer cells. While not wishing to be bound to any particular theory, they propose that Dsg2 can function in the cell to promote EMT.

The authors of international patent publication WO 99/57149 suggest that cell adhesion recognition (CAR) sites derived from the EC2 domain of Dsg2 can be used as modulating agents for treating cancer and/or inhibiting metastasis. The authors state that such modulating agents should inhibit cadherin-mediated cell adhesion. Surprisingly, the inventors have determined that the CAR site within the EC2 domain of Dsg2 does *not* have a function in mediating the EMT promoting activity of Dsg2. In contrast, non-CAR sequences of the EC2 domain of Dsg2 regulate the EMT promoting activity of Dsg2. Peptide fragments derived from non-CAR sequences of the EC2 domain of Dsg2 block EMT and cell invasion *in vitro,* as do antibodies raised to an EC2 domain of Dsg2 without the CAR sequence. Thus, the inventors have demonstrated that non-CAR sequences of the EC2 domain of Dsg2 regulate the EMT promoting function of Dsg2. Until the present invention, no role has been ascribed to non-CAR sequences present in the EC2 domain of Dsg2. The non-CAR sequences have no homology to the CAR sequences.

Peptide fragments derived from non-CAR sequences of the EC2 domain of Dsg2, and antibodies raised to an EC2 domain of Dsg2 without the CAR sequence, antagonise the EMT promoting function of Dsg2. Therefore, antagonists of the function of the non-CAR sequences of the EC2 domain of Dsg2 clearly have much utility as agents for preventing or treating cancer, particularly by reducing EMT and associated invasion and metastatic potential of cancerous cells. This was not known, and could not have been predicated from, any information previously known concerning Dsg2 and its role in EMT.

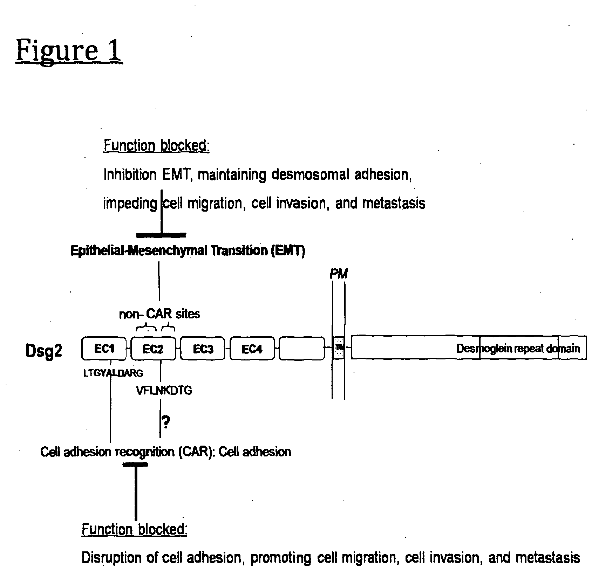

Dsg2 (desmoglein2) is a human transmembrane cell adhesion protein. A schematic overview of the protein structure is provided in Figure 1. An example of an amino acid sequence of Dsg2 is provided at the end of the examples in SEQ ID NO:2; further examples can be located from protein databases, for example NCBI accession NP_001934.2.

The EC2 domain of Dsg2 (extracellular domain 2) is located from amino acid positions 161 to 273 of the sequence provided in SEQ ID NO:2, and is provided below:

QDVFVGSVEELSAAHTLVMKINATDADEPNTLNSKISYRIVSLEPAYPPVFYLNK
DTGEIYTTSVTLDREEHSSYTLTVEARDGNGEVTDKPVKQAQVQIRILDVNDNIP
VVE (SEQ ID NO:3).

The proposed CAR sequence in the EC2 domain of Dsg2 is located from amino acid positions 210 to 218 of the sequence provided in SEQ ID NO:2, and is provided below: VFYLNKDTG (SEQ ID NO:4)

The inventors have identified non-CAR sequences within the EC2 domain of Dsg2 that have a role in regulating the EMT promoting activity of Dsg2. This region is located from amino acid positions 160 to 199 of the sequence provided in SEQ ID NO:2, and is provided below:
TQDVFVGSVEELSAAHTLVMKINATDADEPNTLNSKISYR (SEQ ID NO:1)

The first aspect of the disclosure concerns antagonists of Dsg2, in which the antagonist modulates the function of the amino acid sequence: TQDVFVGSVEELSAAHTLVMKINATDADEPNTLNSKISYR (SEQ ID NO:1), or a fragment or variant thereof, of the EC2 domain of Dsg2.

### By "the amino acid sequence:

TQDVFVGSVEELSAAHTLVMKINATDADEPNTLNSKISYR (SEQ ID NO:1), or a fragment or variant thereof, of the EC2 domain of Dsg2" we include that the antagonist modulates the function of the amino acid sequence of SEQ ID NO:1 to regulate the EMT promoting function of Dsg2. By "a fragment or variant thereof' of SEQ ID NO:1, we include that the antagonist affect part of the EC2 domain defined in SEQ ID NO:1, or variants of that sequence.

A "fragment" of said peptide will preferably comprise less than the total amino acid sequence of the full native peptide; preferably the fragment retains its biological activity: in this case, its ability to regulate the EMT promoting function of Dsg2.

A "variant" of the peptide also refers to a peptide wherein at one or more positions there have been amino acid insertions, deletions, or substitutions, either conservative or non-conservative, provided that such changes result in a protein whose basic properties, for example ability to regulate the EMT promoting function of Dsg2; protein interaction; thermostability; activity in a certain pH-range (pH-stability), have not significantly been changed. "Significantly" in this context means that one skilled in the art would say that the properties of the variant may still be different but would not be unobvious over the ones of the original protein.

By "conservative substitutions" is intended combinations such as Gly, Ala; Val, Ile, Leu; Asp, Glu; Asn, Gln; Ser, Thr; Lys, Arg; and Phe, Tyr.

Such variants may be made using the methods of protein engineering and site-directed mutagenesis as would be well known to those skilled in the art.

The accompanying examples provide assays that can be readily used by the skilled person to measure the effect of Dsg2 on EMT and cell invasion: the "Blocking EMT assay" and the "Cell invasion assay". As shown by the inventors, these assays can be used to measure the antagonistic potential of agents on Dsg2-mediated EMT and cell invasion.

By "antagonist" we include any substance that interferes with the physiological action of Dsg2 polypeptide; preferably by affecting the EMT promoting function. Preferably the aspects of the disclosure provide a therapeutically effective amount of the said antagonist.

For the purposes of the present specification a "therapeutically effective amount" of an antagonist is an amount of such an antagonist that is sufficient to prevent or treat cancer in a subject to whom the antagonist is administered.

The antagonists of the invention reduce the EMT promoting function of Dsg2; preferably by reducing the function of the amino acid sequence: TQDVFVGSVEELSAAHTLVMKINATDADEPNTLNSKISYR (SEQ ID NO:1), or a fragment or variant thereof, of the EC2 domain of Dsg2. This can be measured as a reduction in the EMT promoting function of Dsg2; preferably the
antagonist reduces the function by 10%, 20%, 30%, 40%, 50%, 60%, 70%, 80%, 90%, 95%, 99% or more. The "Blocking EMT assay" and "Cell invasion assay" described in the accompanying examples can be used measure the EMT promoting function of Dsg2, and hence the effect of the antagonist on that activity.

It can be appreciated that there are various ways in which an antagonist can modulate the function of the amino acid sequence: TQDVFVGSVEELSAAHTLVMKINATDADEPNTLNSKISYR (SEQ ID NO:1), or a fragment or variant thereof, of the EC2 domain of Dsg2.

For example, the antagonist may specifically bind the amino acid sequence of SEQ ID NO:1. Such an antagonist can prevent the non-CAR region of EC2 regulating the EMT promoting activity of Dsg2. by "specifically binds to", we include where the antagonist binds to some of the amino acid residues in the amino acid sequence of SEQ ID NO:1, i.e. the antagonist does not have to bind to all the residues to prevent the non-CAR region of EC2 regulating the EMT promoting activity of Dsg2

The antagonist may include some or all of the amino acid sequence of SEQ ID NO:1, or amino acid derivatives or analogues thereof. Such an antagonist can be a competitive inhibitor for the function mediated by the non-CAR region of EC2.

The antagonist may bind to a molecule which the non-CAR region of EC2 interacts with, as part of its function of regulating the EMT promoting activity of Dsg2.

An example of an antagonist would include a chemical ligand that binds to and affects said polypeptide function, and in broader terms this could also include an antibody, or antibody fragment, that binds to one of the said polypeptides such that the polypeptide cannot effect its normal function. The antagonist may also alter the sub-cellular localisation of polypeptide. In this way, the amount of functional polypeptide is reduced.

The use of antibodies as agents to modulate polypeptide activity is well known. Indeed, therapeutic agents based on antibodies are increasingly being used in medicine, including several different types of cancer. In the accompanying examples, the inventors provide exemplification of polyclonal antibody that can bind to a Dsg2 polypeptide lacking a CAR sequence in EC2; hence this antibody is an antagonist according to the second aspect of the invention.

Antibodies may be produced as polyclonal sera by injecting antigen into animals. Preferred polyclonal antibodies may be raised by inoculating an animal (e.g. a rabbit) with antigen using techniques known to the art.

Polyclonal antibodies, for use in treating human subjects, may be raised against a number of peptides derived from the amino acid sequence of SEQ ID NO:1. For instance, as shown in the accompanying examples, a polyclonal antibody was raised to the sequence: TQDVFVGSVEELSAAHTLVMKINATDADEPNTLNSKISYRIVSLEPAYPPVFYLN KDC (SEQ ID NO:5) which corresponds to part of the Dsg2 EC2 domain without a CAR region.

Alternatively the antibody may be monoclonal. Conventional hybridoma techniques may be used to raise such antibodies. The antigen used to generate monoclonal antibodies for use as antagonists in the first aspect of the invention may be the same as would be used to generate polyclonal sera; i.e. all or a fragment or variant of
TQDVFVGSVEELSAAHTLVMKINATDADEPNTLNSKISYRIVSLEPAYPPVFYLN KDC (SEQ ID NO: 5).

In their simplest form, antibodies or immunoglobulin proteins are Y-shaped molecules usually exemplified by the IgG class of antibodies. The molecule consists of four polypeptide chains two identical heavy (H) chains and two identical (L) chains of approximately 50kD and 25kD each respectively. Each light chain is bound to a heavy chain (H-L) by disulphide and non-covalent bonds. Two identical H-L chain combinations are linked to each other by similar non-covalent and disulphide bonds between the two H chains to form the basic four chain immunoglobulin structure (H-L)₂.

Light chain immunoglobulins are made up of one V-domain (V_{L}) and one constant domain (C_{L}) whereas heavy chains consist of one V-domain and, depending on H chain isotype, three or four C-domains (C_{H}1, C_{H}2, C_{H}3 and C_{H}4).

At the N-terminal region of each light or heavy chain is a variable (V) domain that varies greatly in sequence, and is responsible for specific binding to antigen. Antibody specificity for antigen is actually determined by amino acid sequences within the V-regions known as hypervariable loops or Complementarity Determining Regions (CDRs). Each H and L chain V regions possess 3 such CDRs, and it is the combination of all 6 that forms the antibody's antigen binding site. The remaining V-region amino acids which exhibit less variation and which support the hypervariable loops are called frameworks regions (FRs).

The regions beyond the variable domains (C-domains) are relatively constant in sequence. It will be appreciated that the characterising feature of antibodies according to the invention is the V_{H} and V_{L} domains. It will be further appreciated that the precise nature of the C_{H} and C_{L} domains is not, on the whole, critical to the invention. In fact preferred antibodies for use in the invention may have very different C_{H} and C_{L} domains. Furthermore, as discussed more fully below, preferred antibody functional derivatives may comprise the Variable domains without a C-domain (e.g. scFV antibodies).

Preferred antibodies considered to be antagonists according to the invention may have the V_{L} (first domain) and V_{H} (second domain) domains. A derivative thereof may have 75% sequence identity, more preferably 90% sequence identity and most preferably has at least 95% sequence identity. It will be appreciated that most sequence variation may occur in the framework regions (FRs) whereas the sequence of the CDRs of the antibodies, and functional derivatives thereof, should be most conserved.

A number of preferred embodiments of the antagonists of use in the invention relate to molecules with both Variable and Constant domains. However it will be appreciated that antibody fragments (e.g. scFV antibodies or FAbs) are also encompassed by the invention that comprise essentially the Variable region of an antibody without any Constant region.

An scFV antibody fragment considered to be an antagonist of use in the invention may comprise the whole of the V_{H} and V_{L} domains of an antibody raised against SEQ ID NO:1 of Dsg2. The V_{H} and V_{L} domains may be separated by a suitable linker peptide.

Antibodies, and particularly mAbs, generated in one species are known to have several serious drawbacks when used to treat a different species. For instance when murine antibodies are used in humans they tend to have a short circulating half-life in serum and may be recognised as foreign proteins by the immune system of a patient being treated. This may lead to the development of an unwanted human anti-mouse antibody (HAMA) response. This is particularly troublesome when frequent administration of an antibody is required as it can enhance its clearance, block its therapeutic effect, and induce hypersensitivity reactions. These factors limit the use of mouse monoclonal antibodies in human therapy and have prompted the development of antibody engineering technology to generate humanised antibodies.

Therefore, where the antibody is to be used as a therapeutic agent for preventing or treating cancer in a human subject, then it is preferred that antibodies and fragments thereof of non-human source are humanised; such antibodies are considered to be antibody derivatives for the purposes of this invention.

Humanisation may be achieved by splicing V region sequences (e.g. from a monoclonal antibody generated in a non-human hybridoma) with C region (and ideally FRs from V region) sequences from human antibodies. The resulting 'engineered' antibodies are less immunogenic in humans than the non-human antibodies from which they were derived and so are better suited for clinical use.

Humanised antibodies may be chimeric monoclonal antibodies, in which, using recombinant DNA technology, rodent immunoglobulin constant regions are replaced by the constant regions of human antibodies. The chimeric H chain and L chain genes may then be cloned into expression vectors containing suitable regulatory elements and induced into mammalian cells in order to produce fully glycosylated antibodies. By choosing an appropriate human H chain C region gene for this process, the biological activity of the antibody may be pre-determined. Such chimeric molecules may be used to treat or prevent glaucoma.

Further humanisation of antibodies may involve CDR-grafting or reshaping of antibodies. Such antibodies are produced by transplanting the heavy and light chain CDRs of a non-human antibody (which form the antibody's antigen binding site) into the corresponding framework regions of a human antibody.

Humanised antibody fragments represent preferred agents for use according to the invention. Human FAbs recognising an epitope on SEQ ID NO:1 may be identified through screening a phage library of variable chain human antibodies. Techniques known to the art (e.g as developed by Morphosys or Cambridge Antibody Technology) may be employed to generate Fabs that may be used as antagonists according to the invention. In brief a human combinatorial Fab antibody library may be generated by transferring the heavy and light chain variable regions from a single-chain Fv library into a Fab display vector. This library may yield 2.1 x 10¹⁰ different antibody fragments. The peptide may then be used as "bait" to identify antibody fragments from then library that have the desired binding properties.

Domain antibodies (dAbs) represent another preferred agent that may be used according to this embodiment of the invention. dAbs are the smallest functional binding unit of antibodies and correspond to the variable regions of either the heavy or light chains of human antibodies. Such dAbs may have a molecule weight of around 13kDa (corresponding to about 1/10 (or less) the size of a full antibody).

Further preferred agents that may be used according to this embodiment of the invention include bispecific Fab-scFv (a "bibody") and trispecific Fab- (scFv)(2) (a "tribody"). For bibodies or tribodies, a scFv molecule is fused to one or both of the VL-CL (L) and VH-CH₁ (Fd) chains, e.g., to produce a tribody two scFvs are fused to C-term of Fab while in a bibody one scFv is fused to C-term of Fab. The preparation of such molecules can be routinely performed by the skilled person from information available in the field.

In the third and fourth aspects of the invention, the antagonists are peptides or polypeptides comprising some or all of the amino acid sequence of SEQ ID NO:1, or amino acid derivative or analogues thereof.

In the accompanying examples, the inventors provide exemplification of peptides containing some of the amino acid sequence of SEQ ID NO:1: KINATDADEPNTLNSKISYR (SEQ ID NO:6) and EELSAAHTLV (SEQ ID NO:7). The inventors have demonstrated that the peptides can antagonise the EMT promoting function of Dsg2, and hence they are preferred embodiments of the antagonist of the invention.

In addition to the peptides of SEQ ID NO:6 and SEQ ID NO:7, further peptides can be prepared comprising some of the amino acid sequence of SEQ ID NO:1. The utility of such further peptides as antagonists of the EMT promoting function of Dsg2 can be measured using the "Blocking EMT assay" and the "Cell invasion assay" described in the accompanying examples.

The preparation of peptides is a routine process. For example, the peptides used in the accompanying experiments were synthesized by Peptide 2.0 Inc (Chantilly, VA, USA); many other companies offer the commercial synthesis of peptides. Laboratory techniques are also well known for the preparation of peptides, such methods being readily performed by a skilled person. Hence the skilled person can prepare peptides containing some or all of the amino acid sequence of SEQ ID NO:1, and test their utility as antagonists of the EMT promoting function of Dsg2 using the information provided herein and from common general knowledge.

The peptide antagonists according to the invention may comprise all 40 amino acid residues of SEQ ID NO:1, with or without any amino acid substitutions. However, as mentioned above the inventors have determined that peptides having 10 amino acids (EELSAAHTLV (SEQ ID NO:7)) and 20 amino acids (KINATDADEPNTLNSKISYR (SEQ ID NO:6)) can antagonise the EMT promoting function of Dsg2. Accordingly preferred peptides according to the invention can range from about 10 amino acids (for example, 7, 8, 9, 10, 11 or 12) to around 20 amino acids (for example, 18, 19, 20, 21, 22).

The peptide antagonists can also contain further amino acid sequences which are not derived from the amino acid sequence of SEQ ID NO:1: for example, other amino acid sequences which provide a separate function of the peptide (such as a tag, or a catalytic domain). Such peptides are also included in the antagonists of the invention.

Also, it will be appreciated that the invention may be put into effect using derivatives or analogues of these preferred peptides that still lie within the definition of the amino acid sequence provided by SEQ ID NO:1.

By the term "derivative or analogue thereof', we mean a peptide within which amino acids residues are replaced by residues (whether natural amino acids, non-natural amino acids or amino acid mimics) with similar side chains or peptide backbone properties. Additionally, either one or both terminals of such peptides may be protected by N and C-terminal protecting groups, for example, groups with similar properties to acetyl or amide groups. It will be appreciated that the amino acid sequenced may be varied, truncated or modified once the final peptide is formed. As mentioned above, the skilled person can test a derivative or analogue for utility as antagonists of the EMT promoting function of Dsg2 using the information provided herein and from common general knowledge.

Such derivatives may increase or decrease the peptide half-life in vivo. Examples of derivatives capable of increasing the half-life of peptide and polypeptides according to the invention include peptoid derivatives of the polypeptides, D-amino acid derivatives of the polypeptides, and peptide-peptoid hybrids.

Peptides and polypeptides according to the invention may be subject to degradation by a number of means (such as protease activity in biological systems). Such degradation may limit the bioavailability of the polypeptides and hence the ability of the polypeptides to achieve their biological function. There are wide ranges of well-established techniques by which derivatives that have enhanced stability in biological contexts can be designed and produced. Such polypeptide derivatives may have improved bioavailability as a result of increased resistance to protease-mediated degradation. Preferably, a derivative or analogue suitable for use according to the invention is more protease-resistant than the peptide from which it is derived.

Preferably, the polypeptide may be made more protease-resistant by protecting the N and/or C terminal. For example, the N terminal may be protected by an acetyl group, or by an alkyl or aryl group, or an alkyl-CO- or aryl-CO- group, each of which may be optionally substituted. The C terminal may be protected by an amide group or by a substituted amide group.

Protease-resistance of a polypeptide derivative and the polypeptide from which it is derived may be evaluated by means of well-known protein degradation assays. The relative values of protease resistance for the polypeptide derivative and polypeptide may then be compared.

Peptoid derivatives of the polypeptides of the invention may be readily designed from knowledge of the structure of the polypeptide. Commercially available software may be used to develop peptoid derivatives according to well-established protocols.

Retropeptoids, (in which all amino acids are replaced by peptoid residues in reversed order) are also able to mimic antibacterial polypeptides derived from apolipoproteins. A retropeptoid is expected to bind in the opposite direction in the ligand-binding groove, as compared to a peptide or peptoid-peptide hybrid containing one peptoid residue. As a result, the side chains of the peptoid residues are able to point in the same direction as the side chains in the original peptide.

A further embodiment of a modified form of peptides according to the invention comprises D-amino acid forms of the peptide. The preparation of peptides using D-amino acids rather than L-amino acids greatly decreases any unwanted breakdown of such an agent by normal metabolic processes, decreasing the amounts of agent which need to be administered, along with the frequency of its administration.

The peptides, analogues, or derivatives of the invention represent products that may advantageously be expressed by biological cells.

The term "peptidomimetic" refers to a compound that mimics the conformation and desirable features of a particular peptide as a therapeutic agent, but that avoids the undesirable features. For example, morphine is a compound which can be orally administered, and which is a peptidomimetic of the peptide endorphin. There are a number of different approaches to the design and synthesis of peptidomimetics, as is well known in the art.

As well as antagonists which affect the EMT promoting function of Dsg2 by modulating the function of the amino acid sequence: TQDVFVGSVEELSAAHTLVMKINATDADEPNTLNSKISYR (SEQ ID NO:1), another type of antagonist of use in the invention can be a Dsg2 polypeptide in which the amino acid sequence of (SEQ ID NO:1) is altered so as to be non-functional. Such a polypeptide can act as an antagonist by functioning as a competitive inhibitor of native Dsg2, and hence reduce the EMT promoting function of native Dsg2.

Until the present invention, it was not known and was not obvious that the amino acid region of SEQ ID NO:1 regulated the EMT promoting function of Dsg2.

As mentioned above, the skilled person can test whether a Dsg2 polypeptide having one or more amino acid residue replacements, inversion and/or deletions within the sequence TQDVFVGSVEELSAAHTLVMKINATDADEPNTLNSKISYR (SEQ ID NO:1) has utility as antagonists of the EMT promoting function of Dsg2 using the "Blocking EMT assay" and "Cell invasion assay" described in the accompanying examples.

A "variant" of the polypeptide refers to Dsg2 polypeptide wherein at one or more positions there have been amino acid insertions, deletions, or substitutions, either conservative or non-conservative. For example, the polypeptide may contain deletions of some or all of the amino acid residues of SEQ ID NO:1.

Methods of preparing a polypeptide of the invention are well known in the art; for example using recombinant DNA technologies as set out in Sambrook et al (2001): Molecular cloning, a laboratory manual, 3nd edition, Cold Spring Harbor Press, Cold Spring Harbor, New York.

An example of an amino acid sequence of Dsg2 is provided at the end of the examples in SEQ ID NO:2; further examples can be located from protein databases, for example NCBI accession number NP_001934.2. An example of a nucleic acid sequence encoding an amino acid sequence of Dsg2 can be found at NCBI accession number NG_007072.2.

From this information, the skilled person can readily prepare a Dsg2 polypeptide having one or more amino acid residue replacements, inversion and/or deletions within the sequence TQDVFVGSVEELSAAHTLVMKINATDADEPNTLNSKISYR (SEQ ID NO:1).

The antagonist may be a polypeptide comprising the amino acid sequence of SEQ ID NO:9. SEQ ID NO: 9 is provided at the end of the examples below. The polypeptide sequence of SEQ ID NO:9
corresponds to a Dsg2 polypeptide having the amino acid sequence of SEQ ID NO:2, but without the EC1 and EC2 CAR sequences.

The invention provides an antagonist of the EMT promoting function of Dsg2 for use as a medicament for preventing or treating cancer.

As mentioned above, the authors of international patent publication WO 99/57149 suggest that cell adhesion recognition (CAR) sites derived from the EC2 domain of Dsg2 can be used as modulating agents for treating cancer and/or inhibiting metastasis. The authors state that such modulating agents should inhibit cadherin-mediated cell adhesion.

However, we wish to point out that modulating agents derived from the CAR site of the EC2 domain of Dsg2 *do not* function as antagonists of the EMT promoting function of Dsg2. This can be clearly seen from the data presented in the accompanying examples. Hence the modulating agents disclosed in WO 99/57149 are *not* antagonists of the third aspect of the invention.

Moreover, until the present invention, it was not known and was not obvious which region of the Dsg2 polypeptide was responsible for regulating the EMT promoting function of Dsg2.

By "antagonists of the EMT promoting function of Dsg2", we include those antagonists discussed above in relation to the invention; i.e. where the antagonist specifically binds the amino acid sequence of SEQ ID NO:1; where the antagonist includes some or all of the amino acid sequence of SEQ ID NO:1, or amino acid derivatives or analogues thereof; where the antagonist binds to a molecule which the non-CAR region of EC2 interacts with, as part of its function of regulating the EMT promoting activity of Dsg2.

The utility of further agents as antagonists of the EMT promoting function of Dsg2 can be measured using the "Blocking EMT assay" and the "Cell invasion assay" described in the accompanying examples. For example, the antagonist may be identified according to the screening method according to the invention.

As discussed above, the antagonists of the aspects of the invention function to reduce the EMT promoting function of Dsg2. EMT is a program of development of cells characterized by loss of cell adhesion and increased cell mobility. EMT encompasses a number of different developmental processes. Those most relevant to the function of Dsg2 in cancer include cell metastasis and cell invasion. Therefore, preferably the antagonists of the aspects of the invention reduce the cell metastasis and/or cell invasion promoting function of Dsg2. Methods of determining the effect of an antagonist on the cell metastasis and/or cell invasion promoting function of Dsg2 are provided herein in the "Blocking EMT assay" and the "Cell invasion assay" described in the accompanying examples.

By "cancer" we include all types of cancer; for example, bladder; breast (female and male); colon; rectal; endometrial; kidney (renal cell); leukemias; lung; melanoma; Non-Hodgkin lymphoma; pancreatic; prostate; skin; and thyroid.

In the accompanying examples the inventors have demonstrated the EMT promoting function of Dsg2 in prostate cancer, breast cancer and skin cancer cell lines. Hence a preferred embodiment of the invention is wherein said cancer is prostate cancer, breast cancer or skin cancer.

The antagonists of the invention are used to prevent or treat cancer.

Methods of diagnosing cancer are well known in the art; see, for example, http://www.cancer.gov/ which provides details of different types of cancer; how they are diagnosed; and potential treatments.

The antagonists of the invention are used as medicaments. Various means by which the medicaments can be formulated are provided below.

A further aspect of the invention provide the use of an antagonist according to the invention in the manufacture of a medicament for preventing or treating cancer.

Until the present invention, it was not known and was not obvious which region of the Dsg2 polypeptide was responsible for regulating the EMT promoting function of Dsg2. Hence the peptides of the invention could not have been expected to have utility as antagonists of the EMT promoting function of Dsg2.

Further information concerning the preparation of a peptide according to the aspect of the invention may be found above. Also, the example above provides details as to how a peptide of the invention can be prepared.

A further aspect of the invention provides a pharmaceutical composition comprising a therapeutically effective amount of an antibody used according to the invention and optionally a pharmaceutically acceptable vehicle. In one embodiment, the amount of the antibody is an amount from about 0.01 mg to about 800 mg. In another embodiment, the amount of the antibody is an amount from about 0.01 mg to about 500 mg. In another embodiment, the amount of the antibody is an amount from about 0.01 mg to about 250 mg. In another embodiment, the amount of the antibody is an amount from about 0.1 mg to about 60 mg. In another embodiment, the amount of the antibody is an amount from about 0.1 mg to about 20 mg.

This disclosure provides a process for making a pharmaceutical composition comprising combining a therapeutically effective amount of an antibody as used according to the invention and a pharmaceutically acceptable vehicle. A "therapeutically effective amount" is any amount of an antibody which, when administered to a subject provides prevention and/or treatment of cancer. A "subject" is a vertebrate, mammal, domestic animal or human being.

A "pharmaceutically acceptable vehicle" as referred to herein is any physiological vehicle known to those of ordinary skill in the art useful in formulating pharmaceutical compositions.

There are a number of different ways in which the antagonists for use in the invention can be used as a medicament.

The antagonists may be combined in compositions having a number of different forms depending, in particular on the manner in which the composition is to be used. Thus, for example, the composition may be in the form of a powder, tablet, capsule, liquid, ointment, cream, gel, hydrogel, aerosol, spray, micelle, transdermal patch, liposome or any other suitable form that may be administered to a person or animal. It will be appreciated that the vehicle of the composition of the invention should be one which is well tolerated by the subject to whom it is given, and preferably enables delivery of the antagonist to the target cell, tissue, or organ. Hence, it is preferred that that antagonist is delivered by means of a suitably protected carrier particle, for example, a micelle.

The antibodies, or functional derivatives thereof, may be used in a number of ways. For instance, systemic administration may be required in which case the antibodies or derivatives thereof may be contained within a composition which may, for example, be ingested orally in the form of a tablet, capsule or liquid. It is preferred that the antibodies, or derivatives thereof, are administered by injection into the blood stream. Injections may be intravenous (bolus or infusion) or subcutaneous (bolus or infusion). Alternatively the antibodies may be injected directly to the liver.

Polypeptide therapeutic entities may be combined in pharmaceutical compositions having a number of different forms depending, in particular on the manner in which the composition is to be used. Thus, for example, the composition may be in the form of a powder, tablet, capsule, liquid, ointment, cream, gel, hydrogel, aerosol, spray, micelle, transdermal patch, liposome or any other suitable form that may be administered to a person or animal. It will be appreciated that the vehicle of the composition of the invention should be one which is well tolerated by the subject to whom it is given, and preferably enables delivery of the therapeutic to the target cell, tissue, or organ.

In a preferred embodiment, the pharmaceutical vehicle is a liquid and the pharmaceutical composition is in the form of a solution. In another embodiment, the pharmaceutical vehicle is a gel and the composition is in the form of a cream or the like.

Antagonists may also be incorporated within a slow or delayed release device. Such devices may, for example, be inserted on or under the skin, and the compound may be released over weeks or even months. Such devices may be particularly advantageous when long term treatment with an antagonist according to the invention is required and which would normally require frequent administration (e.g. at least daily injection).

It will be appreciated that the amount of an antagonist that is required is determined by its biological activity and bioavailability which in turn depends on the mode of administration, the physicochemical properties of the antagonist employed, and whether the antagonist is being used as a monotherapy or in a combined therapy. Also, the amount will be determined by the number and state of target cells to be treated. The frequency of administration will also be influenced by the above-mentioned factors and particularly the half-life of the antagonist within the subject being treated.

Optimal dosages to be administered may be determined by those skilled in the art, and will vary with the particular antagonist in use, the strength of the preparation, the mode of administration, and the advancement of the disease condition. Additional factors depending on the particular subject being treated will result in a need to adjust dosages, including subject age, weight, gender, diet, and time of administration.

Known procedures, such as those conventionally employed by the pharmaceutical industry (e.g. *in vivo* experimentation, clinical trials, etc.), may be used to establish specific formulations of antagonist according to the invention and precise therapeutic regimes (such as daily doses of the antagonist and the frequency of administration).

Generally, a daily dose of between 0.01 µg/kg of body weight and 0.5 g/kg of body weight of antagonist according to the invention may be used for the prevention and/or treatment of cancer, depending upon which specific antagonist is used. More preferably, the daily dose is between 0.01 mg/kg of body weight and 200 mg/kg of body weight, and most preferably, between approximately 1mg/kg and 100 mg/kg.

Daily doses may be given as a single administration (e.g. a single daily injection). Alternatively, the antagonist used may require administration twice or more times during a day. As an example, an antagonist according to the invention may be administered as two (or more depending upon the severity of the condition) daily doses of between 25 mg and 7000 mg (i.e. assuming a body weight of 70kg). A patient receiving treatment may take a first dose upon waking and then a second dose in the evening (if on a two dose regime) or at 3 or 4 hourly intervals thereafter. Alternatively, a slow release device may be used to provide optimal doses to a patient without the need to administer repeated doses.

An aspect of the disclosure provides a method of screening for antagonists of Dsg2, wherein said antagonist modulates the function of the amino acid sequence: TQDVFVGSVEELSAAHTLVMKINATDADEPNTLNSKISYR (SEQ ID NO:1), or a fragment or variant thereof, of the EC2 domain of Dsg2, comprising (i) exposing a cell having a Dsg2 polypeptide to a test compound; (ii) and the effect of the test compound on the function of the amino acid sequence of SEQ ID NO:1 of the EC2 domain of Dsg2 is determined.

The method can be used to identify compounds which may be of use in treating cancer.

All of the features described herein (including any accompanying claims, abstract and drawings), and/or all of the steps of any method or process so disclosed, may be combined with any of the above aspects in any combination, except combinations where at least some of such features and/or steps are mutually exclusive.

The invention will now be further described with reference to the following examples and Figures.
**Figure 1****:** The difference in functional blocking between CAR and non-CAR sites.
**Figure 2****:** Anti-Dsg2 EC2 antibodies inhibit EMT. Anti-EC 1 and anti-EC4 antibodies don't inhibit EMT.
**Figure 3****:** Anti-Dsg2 EC2 antibodies inhibit the invasion abilities of (A) human melanoma A375 cells, (B) human breast cancer MCF7 cells, and (C) human prostate cancer LNCaP cells.
**Figure 4****:** Dsg2 EC2 peptide sequences without its full CAR domain (VFYLNKDTG) were used to generate a rabbit polyclonal antibody. (A) EMT is significantly inhibited by the antibodies generated; (B) The cell invasion assay measures the ability of cells to grow and penetrate a gel made of 'Matrigel'. The antibodies generated inhibit the invasion abilities of human breast cancer MCF7 cells.
**Figure 5****:** 8 synthetic peptides were prepared. (A) EMT is significantly blocked by the non-CAR sequences of Dsg2 EC2, Peptides 4 and 7; whereas the CAR sequences and other non-CAR sequences do not affect EMT. (B) The cell invasion ability of human breast cancer MCF7 cells has been inhibited by the non-CAR sequences, Peptides 4 and 7.

### Example 1: Determining the function of Dsg2 for regulating epithelial-mesenchymal transition.

### Introduction

The present invention concerns inhibition of epithelial-mesenchymal transition (EMT) by modulating the function of non-CAR sequences of the EC2 domain of Dsg2.

Desmosomes are one of the principal types of cell-cell adhesion junction between epithelial, myocardial and other tissues. Such desmosomes contain transmembrane glycoproteins called desmosomal cadherin, desmocollin (Dsc) and desmoglein (Dsg). Each occurs as at least three distinct genetic isoforms that show tissue-specific expression patterns.

Dsg2 is ubiquitously expressed in all tissues that form desmosomes. The extracellular domains of Dsg2 contain four cadherin repeat domains (EC1-4) about 110 amino acids each in length. The extracellular repeat domain EC1 contains cell adhesion recognition (CAR) sites, which provide cell-cell adhesion. Therefore, Dsg2 has been identified to be a transmembrane cell adhesion molecule. However, very recent studies show that Dsg2 is not just a simple cell-cell adhesion molecule. Dsg2 is involved in promotion of angiogenesis, signalling of apoptosis, and is a substrate for MMPs. Moreover, it has previously been determined that Dsg2 can play a role in EMT. EMT is a very different physiological action to cell-cell adhesion.

Against this background, the inventors investigated the role of Dsg2 and specific regions within that protein in regulating EMT, and whether Dsg2 could be modulated to inhibit cancer cell invasion.

### Results and Discussion

It has previously been suggested that Dsg2 has a role in EMT. However it is not known how this activity of Dsg2 is mediated.

Figure 1 presents a schematic diagram of Dsg2, along with known and proposed functions of domains within that protein. The extracellular repeat domain EC1 and EC2 contains cell adhesion recognition (CAR) sites, which provide cell-cell adhesion. The cell adhesion function of Dsg2 CAR sequence (LTGYALDARG; SEQ ID NO: 10) in EC1 domain is known, and it has been proposed that a CAR sequence (VFYLNKDTGE; SEQ ID NO: 4) in EC2 domain has a similar function in cell adhesion.

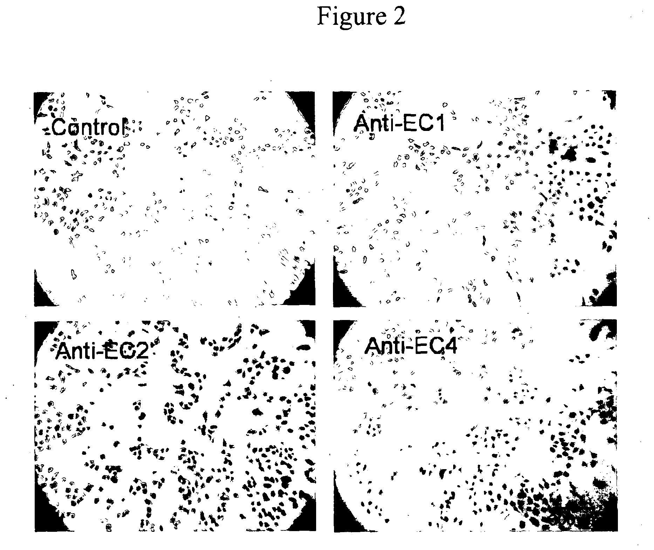

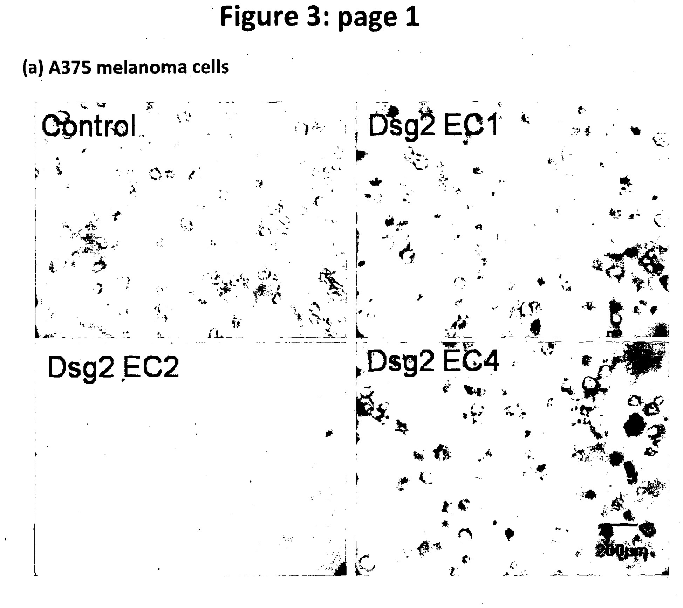

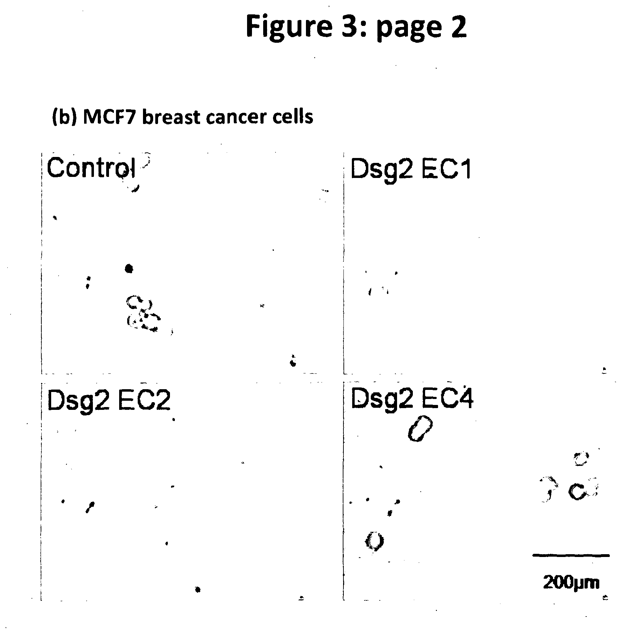

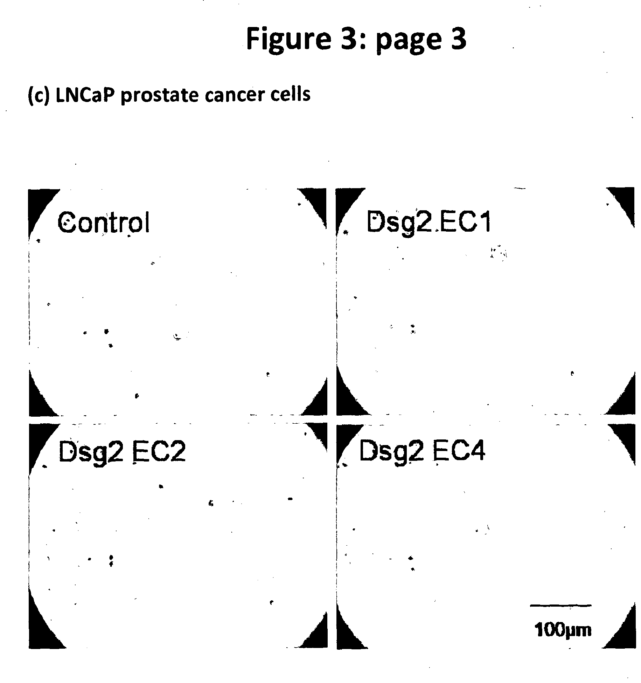

The inventors studied the effect of antibodies that specifically bind to the EC1, EC2 and EC4 domains of Dsg2 on EMT. As can be seen in Figures 2 and 3, Anti-EC2 antibodies inhibit EMT and inhibit cell invasion of human melanoma, breast cancer and prostate cancer cells, while anti-EC1 and anti-EC4 antibodies do not have such an effect. Hence it seems that the EMT regulating activity of Dsg2 is mediated by sequences within the EC2 domain of that protein.

The EC2 domain of Dsg2 has been proposed to contain a CAR domain. CAR domains in Dsg2 and other desmocollin (Dsc) and desmoglein proteins (Dsc1, Dsc2, Dsc3, Dsg1, Dsg2, and Dsg3) have previously been demonstrated to have function in cell adhesion. However, it is known that blocking desmosomal adhesion via CAR *promotes* cell migration, cell invasion, and metastasis. In contrast anti-EC2 antibodies inhibit EMT, resulting in maintaining cell adhesion and impeding cell migration, cell invasion, and metastasis. Hence it seems unlikely that a CAR sequence in the EC2 domain of Dsg2 is responsible for any EMT-promoting activity of that protein.

Accordingly, the inventors decided to locate the region of the EC2 domain of Dsg2 which regulates the EMT promoting activity of that protein. To do so, the inventors generated a rabbit polyclonal antibody to peptide derived from Dsg2 EC2, but lacking the full CAR domain:

TQDVFVGSVEELSAAHTLVMKINATDADEPNTLNSKISYRIVSLEPAYPPVFYLN
KDC (SEQ ID NO:5).

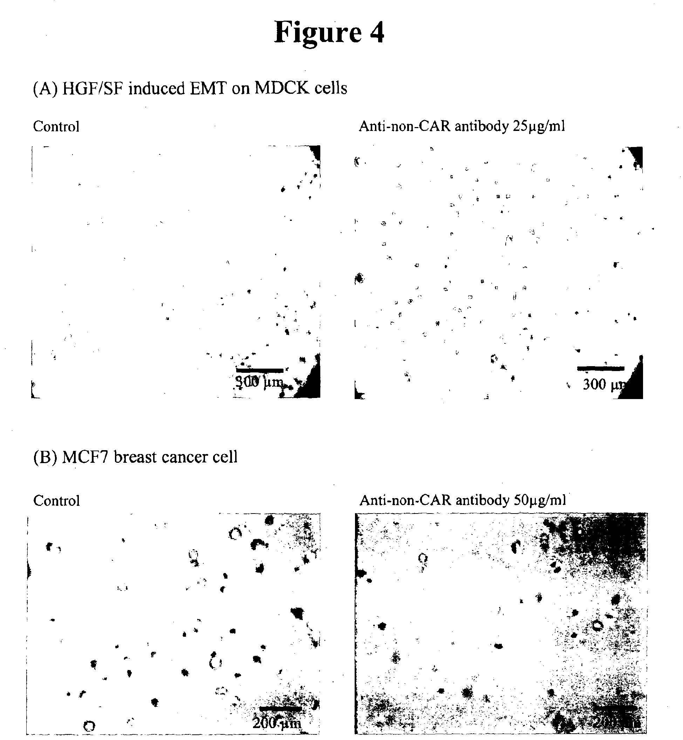

The antibodies were then used in a "blocking EMT assay" and "cell invasion assay" (details below) to determine their effect on cells. The results are shown in Figure 4. It can be seen that EMT is significantly inhibited by the antibodies generated (panel A), and also that the antibodies generated inhibit the invasion abilities of human breast cancer MCF7 cells (panel B). Hence this data clearly shows that the EMT promoting function of Dsg2 is mediated by a non-CAR region in EC2.

The inventors then prepared a series of peptides derived from the EC2 domain of Dsg2:
Peptide 1: Control Peptide (YTRLGANLAG) (SEQ ID NO:11).
Peptide 2: CAR Sequence of EC1 (LTGYALDARG) (SEQ ID NO:10).
Peptide 3: CAR Sequence of EC2 (VFYLNKDTGE) (SEQ ID NO:4).
Peptide 4: non-CAR Sequence of EC2 (KINATDADEPNTLNSKISYR) (SEQ ID NO:6).
Peptide 5: non-CAR Sequence of EC2 (YTTSVTLDREEHSSY) (SEQ ID NO:12).
Peptide 6: non-CAR Sequence of EC2 (ARDGNGEVTDKPVKQ) (SEQ ID NO:13).
Peptide 7: non-CAR Sequence of EC2 (EELSAAHTLV) (SEQ ID NO:7).
Peptide 8: non-CAR Sequence of EC2 (IVSLEPAYPP) (SEQ ID NO: 14).

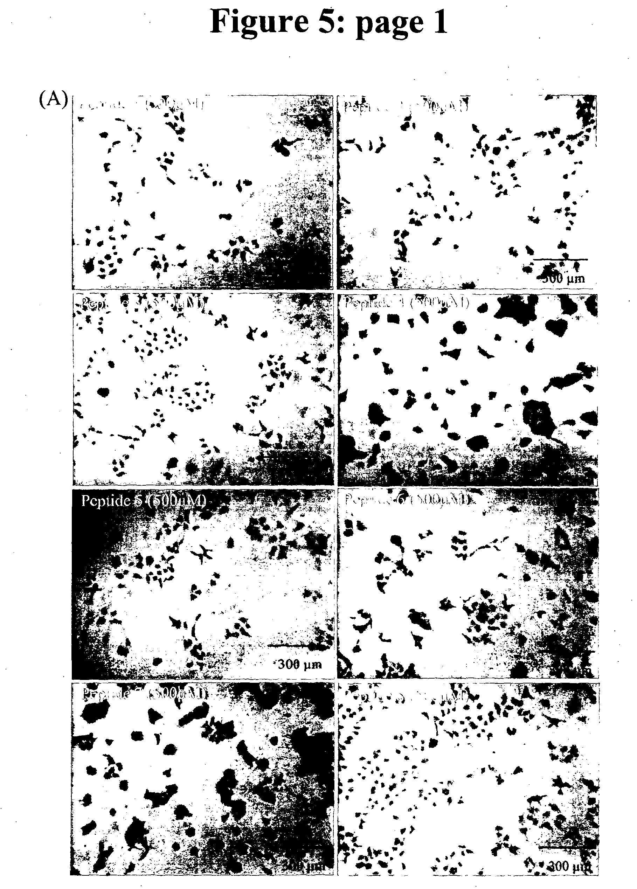

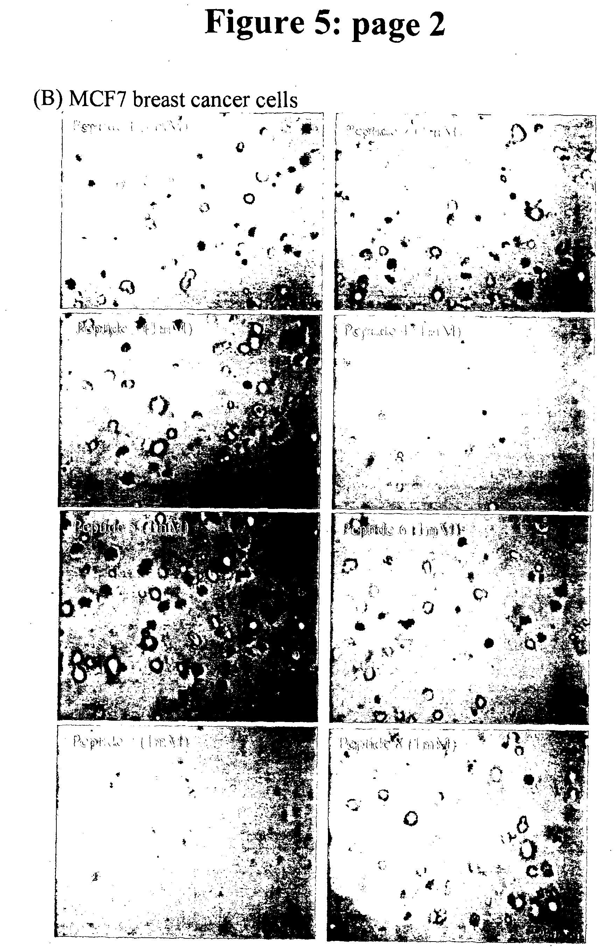

The peptides were then used in a "blocking EMT assay" and "cell invasion assay" (details below) to determine their effect on cells. The results are shown in Figure 5. It can be seen that EMT and cell invasion is significantly inhibited by peptides 4 and 7. Again this data clearly shows that the EMT promoting function of Dsg2 is mediated by a non-CAR region in EC2.

Hence the inventors have demonstrated that inhibition and antagonists of the non-CAR sequences present in EC2 of Dsg2 can block EMT and invasion *in vitro,* and have the potential to block cancer invasion and metastasis *in vivo.*

### Experimental protocols

**Generation of the antibodies:** The polyclonal antibodies were generated by Abgent (San Diego, CA, USA). In brief, the immunizing peptide sequence (TQDVFVGSVEELSAAHTLVMKINATDADEPNTLNSKISYRIVSLEPAYPPVFYLN KDC; SEQ ID NO:5) was synthesized, conjugated with a carrier protein KLH (Keyhole Limpet Hemocyanin) and used in immunization of rabbits. The rabbits were bled and the antibodies were prepared by saturated ammonium sulfate precipitation followed by dialysis against PBS.

**Peptides:** The peptides used in the experiments were synthesized by Peptide 2.0 Inc (Chantilly, VA, USA). Peptide 1: Control Peptide (YTRLGANLAG; SEQ ID NO:11). Peptide 2: CAR sequence of EC1 (LTGYALDARG; SEQ ID NO:10). Peptide 3: CAR sequence of EC2 (VFYLNKDTGE; SEQ ID NO:4). Peptide 4: non-CAR sequence of EC2 (KINATDADEPNTLNSKISYR; SEQ ID NO:6). Peptide 5: non-CAR sequence of EC2 (YTTSVTLDREEHSSY; SEQ ID NO:12). Peptide 6: non-CAR sequence of EC2 (ARDGNGEVTDKPVKQ; SEQ ID NO:13). Peptide 7: non-CAR sequence of EC2 (EELSAAHTLV; SEQ ID NO: 7). Peptide 8: non-CAR sequence of EC2 (IVSLEPAYPP; SEQ ID NO:14).

**Blocking EMT assay:** Antibodies or peptides were added to 10% sub-confluent MDCK cells cultured in standard medium with 2.5ng/ml HGF/SF in 96-well plates for 24h. EMT was induced by HGF/SF in MDCK cells without any antibody or peptide treatment. The effects of different antibodies and peptides in blocking EMT were investigated by adding different concentrations of them to 10% sub-confluent MDCK cells cultured in standard medium with 2.5ng/ml HGF/SF.

**Cell invasion assay:** Growth Factor-Reduced MatrigelTM Matrix (BD, Becton Dickinson Biosciences, Bedford, MA, USA) was thawed in the fridge one day prior to its use. The Matrigel matrix was mixed by pre-cooled pipettes to homogeneity on ice. Four µl of the cell suspension (1×106cells/ml) in standard medium without (Control) or with 1µl different concentrations of antibody or peptide was mixed with 95µl of Matrigel matrix in a cold Eppendorf tube. After thorough mixing using cooled pipettes, 30µl of the cells in the Matrigel mixture was added to 48-well plates. The plates were incubated at 37°C in a CO2 incubator for 1h to polymerize the droplet mixtures. 250µl of standard medium was added to cover the cell/Matrigel droplet and incubated for several days. Blindly-selected cell invasion images were recorded in four areas within a cell/Matrigel droplet by a Nikon 4500 Coolpix digital camera under the microscope.

### Sequence listing

Non-CAR sequence of EC2 of Deg2 (SEQ ID NO: 1)
   TQDVFVGSVEELSAAHTLVMKINATDADEPNTLNSKISYR
Human Dsg2 (SEQ ID NO:2):
EC2 domain of Dsg2 (SEQ ID NO:3) 
QDVFVGSVEELSAAHTLVMKINATDADEPNTLNSKISYRIVSLEPAYPPVFYLNKDTGEIYTTSVTLDREE
HSSYTLTVEARDGNGEVTDKPVKQAQVQIRILDVNDNIPVVE
CAR sequence of EC2 of Dsg2 (SEQ ID NO:4)
   VFYLNKDTGE
Dsg2 EC2 domain without a CAR sequence (SEQ ID NO:5)
   TQDVFVGSVEELSAAHTLVMKINATDADEPNTLNSKISYRIVSLEPAYPPVFYLNKDC
Fragment of non-CAR sequence of EC2 of Dsg2 (SEQ ID NO:6)
   KINATDADEPNTLNSKISYR
Fragment of non-CAR sequence of EC2 of Dsg2 (SEQ ID NO:7)
   EELSAAHTLV
Human Dsg2 without the non-CAR region of EC2 (SEQ ID NO:8): 
marspgraya lllllicfnv gsglhlqvls trnenkllpk hphlvrqkra witapvalre
gedlskknpi akihsdlaee rglkitykyt gkgiteppfg ifvfnkdtge lnvtsildre
etpfflltgy aldargnnve kplelrikvl dindnepvfi vslepayppv fylnkdtgei
yttsvtldre ehssytltve ardgngevtd kpvkqaqvqi rildvndnip vvenkvlegm
veenqvnvev trikvfdade igsdnwlanf tfasgneggy fhietdaqtn egivtlikev
dyeemknldf svivankaaf hksirskykp tpipikvkvk nvkegihfks svisiyvses
mdrsskgqii gnfqafdedt glpaharyvk ledrdnwisv dsvtseikla klpdfesryv
qngtytvkiv aisedyprkt itgtvlinve dindncptli epvqtichda eyvnvtaedl
dghpnsgpfs fsvidkppgm aekwkiarqe stsvllqqse kklgrseiqf lisdnqgfsc
pekqvltltv ceclhgsgcr eaqhdsyvgl gpaaialmil aflllllvpl lllmchcgkg
akgftpipgt iemlhpwnne gappedkvvp sflpvdqggs lvgrngvggm akeatmkgss
sasivkgqhe msemdgrwee hrsllsgrat qftgatgaim ttettktara tgasrdmaga
qaaavalnee flrnyftdka asyteedenh takdcllvys qeeteslnas igccsfiege
lddrflddlg lkfktlaevc lgqkidinke ieqrqkpate tsmntashsl ceqtmvnsen
tyssgssfpv pkslqeanae kvtqeivter svssrqaqkv atplpdpmas rnviatetsy
vtqstmpptt vilgpsqpqs livtervyap astlvdqpya negtvvvter viqphgggsn
plegtqhlqd vpyvmvrere sflapssgvq ptlampniav gqnvtvterv lapastlqss
yqiptensmt arnttvsgag vpgplpdfgl eesghsnsti ttsstrvtkh stvqhsys
Dsg2 without EC1 and EC2 CAR sequence (SEQ ID NO:9) 
marspgraya lllllicfnv gsglhlqvls trnenkllpk hphlvrqkra witapvalre
gedlskknpi akihsdlaee rglkitykyt gkgiteppfg ifvfnkdtge lnvtsildre
etpffl nnve kplelrikvl dindnepvft qdvfvgsvee lsaahtlvmk
inatdadepn tlnskisyri vslepayppi yttsvtldre ehssytltve ardgngevtd
kpvkqaqvqi rildvndnip vvenkvlegm veenqvnvev trikvfdade igsdnwlanf
tfasgneggy fhietdaqtn egivtlikev dyeemknldf svivankaaf hksirskykp
tpipikvkvk nvkegihfks svisiyvses mdrsskgqii gnfqafdedt glpaharyvk
 
ledrdnwisv dsvtseikla klpdfesryv qngtytvkiv aisedyprkt itgtvlinve
dindncptli epvqtichda eyvnvtaedl dghpnsgpfs fsvidkppgm aekwkiarqe
stsvllqqse kklgrseiqf lisdnqgfsc pekqvltltv ceclhgsgcr eaqhdsyvgl
gpaaialmil aflllllvpl lllmchcgkg akgftpipgt iemlhpwnne gappedkvvp
sflpvdqggs lvgrngvggm akeatmkgss sasivkgqhe msemdgrwee hrsllsgrat
qftgatgaim ttettktara tgasrdmaga qaaavalnee flrnyftdka asyteedenh
takdcllvys qeeteslnas igccsfiege lddrflddlg lkfktlaevc lgqkidinke
ieqrqkpate tsmntashsl ceqtmvnsen tyssgssfpv pkslqeanae kvtqeivter
svssrqaqkv atplpdpmas rnviatetsy vtgstmpptt vilgpsqpqs livtervyap
astlvdqpya negtvvvter viqphgggsn plegtqhlqd vpyvmvrere sflapssgvq
ptlampniav gqnvtvterv lapastlqss yqiptensmt arnttvsgag vpgplpdfgl
eesghsnsti ttsstrvtkh stvqhsys
CAR sequence of EC1 of Dsg2 (SEQ ID NO:10)
   LTGYALDARG
Control peptide sequence (SEQ ID NO:11)
   YTRLGANLAG
Fragment of non-CAR sequence of EC2 of Dsg2 (SEQ ID NO:12)
   YTTSVTLDREEHSSY
Fragment of non-CAR sequence of EC2 of Dsg2 (SEQ ID NO:13)
   ARDGNGEVTDKPVKQ
Fragment of non-CAR sequence of EC2 of Dsg2 (SEQ ID NO: 14)
   IVSLEPAYPP

## Claims

1. A monoclonal antibody as an antagonist for use as a medicament for preventing or treating cancer, **characterised in that** the monoclonal antibody specifically binds to the amino acid sequence: TQDVFVGSVEELSAAHTLVMKINATDADEPNTLNSKISYR (SEQ ID NO:1), KINATDADEPNTLNSKISYR (SEQ ID NO:6) or EELSAAHTLV (SEQ ID NO:7) of the non-cell adhesion recognition (CAR) sites region of the EC2 domain of Dsg2 and inhibits epithelial mesenchymal transition (EMT).

2. A monoclonal antibody for use of claim 1 wherein the monoclonal antibody is raised against the amino acid sequence TQDVFVGSVEELSAAHTLVMKINATDADEPNTLNSKISYR (SEQ ID NO:1), KINATDADEPNTLNSKISYR (SEQ ID NO:6) or EELSAAHTLV (SEQ ID NO:7).

3. A polyclonal antibody as an antagonist for use as a medicament for preventing or treating cancer, **characterised in that** the polyclonal antibody is raised against the amino acid sequence TQDVFVGSVEELSAAHTLVMKINATDADEPNTLNSKISYR (SEQ ID NO:1) or SEQ ID NO:5 and inhibits epithelial mesenchymal transition (EMT).

4. The monoclonal antibody or polyclonal antibody for use of any one of claims 1 to 3 wherein said cancer is prostate cancer, breast cancer or skin cancer.

5. The monoclonal antibody or polyclonal antibody for use of any one of claims 1 to 4 wherein the monoclonal antibody or polyclonal antibody reduces the cell metastasis and/or cell invasion promoting function of Dsg2.

6. An isolated polypeptide comprising
(a) the amino acid sequence TQDVFVGSVEELSAAHTLVMKINATDADEPNTLNSKISYR (SEQ ID NO:1), KINATDADEPNTLNSKISYR (SEQ ID NO:6) or EELSAAHTLV (SEQ ID NO:7) wherein said amino acid sequence is capable of inhibiting the epithelial mesenchymal transition (EMT) promoting function of Dsg2; or
(b) a biologically active fragment of the amino acid sequence TQDVFVGSVEELSAAHTLVMKINATDADEPNTLNSKISYR (SEQ ID NO:1), KINATDADEPNTLNSKISYR (SEQ ID NO:6) or EELSAAHTLV (SEQ ID NO:7) wherein said fragment is capable of inhibiting the epithelial mesenchymal transition (EMT) promoting function of Dsg2
for use as a medicament for preventing or treating cancer.

7. An isolated polypeptide comprising
(a) the amino acid sequence SEQ ID NO:9 wherein said amino acid sequence is capable of inhibiting the epithelial mesenchymal transition (EMT) promoting function of Dsg2
for use as a medicament for preventing or treating cancer.

8. The isolated polypeptide for use of claim 6, wherein the polypeptide comprises the amino acid sequence SEQ ID NO:1.

9. The isolated polypeptide for use of claim 6, wherein the polypeptide comprises the amino acid sequence SEQ ID NO:6.

10. The isolated polypeptide for use of claim 6, wherein the polypeptide comprises the amino acid sequence SEQ ID NO:7.

11. The isolated polypeptide for use of any one of claims 6 to 10 wherein the polypeptide reduces the cell metastasis and/or cell invasion promoting function of Dsg2.

12. The isolated polypeptide for use of any one of claims 6 to 11 wherein said cancer is prostate cancer, breast cancer or skin cancer.

13. A pharmaceutical preparation comprising:
(i) a monoclonal antibody which specifically binds to the amino acid sequence: TQDVFVGSVEELSAAHTLVMKINATDADEPNTLNSKISYR (SEQ ID NO:1), KINATDADEPNTLNSKISYR (SEQ ID NO:6) or EELSAAHTLV (SEQ ID NO:7) of the non-cell adhesion recognition (CAR) sites region of the EC2 domain of Dsg2 and inhibits epithelial mesenchymal transition (EMT); or
(ii) a polyclonal antibody raised against the amino acid sequence TQDVFVGSVEELSAAHTLVMKINATDADEPNTLNSKISYR (SEQ ID NO:1) or SEQ ID NO:5 which inhibits epithelial mesenchymal transition (EMT);
and a pharmaceutically acceptable excipient.

14. Use of a monoclonal antibody as defined in any one of claims 1, 2, 4 or 5 in the preparation of a medicament for preventing or treating cancer.

15. Use of a polyclonal antibody as defined in any one of claims 3, 4 or 5 in the preparation of a medicament for preventing or treating cancer.

16. Use of an isolated polypeptide as defined in claim 6 in the preparation of a medicament for preventing or treating cancer.

17. Use of an isolated polypeptide as defined in any one of claims 7 to 10 in the preparation of a medicament for preventing or treating cancer.

## Patentansprüche

1. Monoklonaler Antikörper als Antagonist zur Verwendung als Medikament zur Verhütung oder Behandlung von Krebs, **dadurch gekennzeichnet, dass** der monoklonale Antikörper an die Aminosäuresequenz: TQDVFVGSVEELSAAHTLVMKINATDADEPNTLNSKISYR (SEQ ID NO:1), KINATDADEPNTLNSKISYR (SEQ ID NO:6) oder EELSAAHTLV (SEQ ID NO:7) der Nicht-Zelladhäsionserkennungs- (CAR) Stellen-Region der EC2-Domäne von Dsg2 spezifisch bindet und epithelial-mesenchymale Transition (EMT) hemmt.

2. Monoklonaler Antikörper zur Verwendung nach Anspruch 1, wobei der monoklonale Antikörper gegen die Aminosäuresequenz TQDVFVGSVEELSAAHTLVMKINATDADEPNTLNSKISYR (SEQ ID NO:1), KINATDADEPNTLNSKISYR (SEQ ID NO:6) oder EELSAAHTLV (SEQ ID NO:7) erzeugt ist.

3. Polyklonaler Antikörper als Antagonist zur Verwendung als Medikament zur Verhütung oder Behandlung von Krebs, **dadurch gekennzeichnet, dass** der polyklonale Antikörper gegen die Aminosäuresequenz TQDVFVGSVEELSAAHTLVMKINATDADEPNTLNSKISYR (SEQ ID NO:1) oder SEQ ID NO:5 erzeugt ist und epithelial-mesenchymale Transition (EMT) hemmt.

4. Monoklonaler Antikörper oder polyklonaler Antikörper zur Verwendung nach einem der Ansprüche 1 bis 3, wobei der Krebs Prostatakrebs, Brustkrebs oder Hautkrebs ist.

5. Monoklonaler Antikörper oder polyklonaler Antikörper zur Verwendung nach einem der Ansprüche 1 bis 4, wobei der monoklonale Antikörper oder polyklonale Antikörper die Zellmetastasierung und/oder Zellinvasion fördernde Funktion von Dsg2 reduziert.

6. Isoliertes Polypeptid, umfassend
(a) die Aminosäuresequenz TQDVFVGSVEELSAAHTLVMKINATDADEPNTLNSKISYR (SEQ ID NO:1), KINATDADEPNTLNSKISYR (SEQ ID NO:6) oder EELSAAHTLV (SEQ ID NO:7), wobei die Aminosäuresequenz fähig ist, die epithelial-mesenchymale Transition (EMT) fördernde Funktion von Dsg2 zu hemmen; oder
(b) ein biologisch aktives Fragment der Aminosäuresequenz TQDVFVGSVEELSAAHTLVMKINATDADEPNTLNSKISYR (SEQ ID NO:1), KINATDADEPNTLNSKISYR (SEQ ID NO:6) oder EELSAAHTLV (SEQ ID NO:7), wobei das Fragment fähig ist, die epithelial-mesenchymale Transition (EMT) fördernde Funktion von Dsg2 zu hemmen,
zur Verwendung als Medikament zur Verhütung oder Behandlung von Krebs.

7. Isoliertes Polypeptid, umfassend
(a) die Aminosäuresequenz SEQ ID NO:9, wobei die Aminosäuresequenz fähig ist, die epithelial-mesenchymale Transition (EMT) fördernde Funktion von Dsg2 zu hemmen,
zur Verwendung als Medikament zur Verhütung oder Behandlung von Krebs.

8. Isoliertes Polypeptid zur Verwendung nach Anspruch 6, wobei das Polypeptid die Aminosäuresequenz SEQ ID NO:1 umfasst.

9. Isoliertes Polypeptid zur Verwendung nach Anspruch 6, wobei das Polypeptid die Aminosäuresequenz SEQ ID NO:6 umfasst.

10. Isoliertes Polypeptid zur Verwendung nach Anspruch 6, wobei das Polypeptid die Aminosäuresequenz SEQ ID NO:7 umfasst.

11. Isoliertes Polypeptid zur Verwendung nach einem der Ansprüche 6 bis 10, wobei das Polypeptid die Zellmetastasierung und/oder Zellinvasion fördernde Funktion von Dsg2 reduziert.

12. Isoliertes Polypeptid zur Verwendung nach einem der Ansprüche 6 bis 11, wobei der Krebs Prostatakrebs, Brustkrebs oder Hautkrebs ist.

13. Pharmazeutische Zubereitung, umfassend:
(i) einen monoklonalen Antikörper, der an die Aminosäuresequenz: TQDVFVGSVEELSAAHTLVMKINATDADEPNTLNSKISYR (SEQ ID NO:1), KINATDADEPNTLNSKISYR (SEQ ID NO:6) oder EELSAAHTLV (SEQ ID NO:7) der Nicht-Zelladhäsionserkennungs- (CAR) Stellen-Region der EC2-Domäne von Dsg2 spezifisch bindet und epithelial-mesenchymale Transition (EMT) hemmt; oder
(ii) einen gegen die Aminosäuresequenz TQDVFVGSVEELSAAHTLVMKINATDADEPNTLNSKISYR (SEQ ID NO:1) oder SEQ ID NO:5 erzeugten polyklonalen Antikörper, der epithelial-mesenchymale Transition (EMT) hemmt;
und einen pharmazeutisch verträglichen Hilfsstoff.

14. Verwendung eines monoklonalen Antikörpers, gemäß Definition in einem der Ansprüche 1, 2, 4 oder 5, bei der Herstellung eines Medikaments zur Verhütung oder Behandlung von Krebs.

15. Verwendung eines polyklonalen Antikörpers, gemäß Definition in einem der Ansprüche 3, 4 oder 5, bei der Herstellung eines Medikaments zur Verhütung oder Behandlung von Krebs.

16. Verwendung eines isolierten Polypeptids, gemäß Definition in Anspruch 6, bei der Herstellung eines Medikaments zur Verhütung oder Behandlung von Krebs.

17. Verwendung eines isolierten Polypeptids, gemäß Definition in einem der Ansprüche 7 bis 10, bei der Herstellung eines Medikaments zur Verhütung oder Behandlung von Krebs.

## Revendications

1. Anticorps monoclonal en tant qu'antagoniste pour une utilisation en tant que médicament pour la prévention ou le traitement d'un cancer, **caractérisé en ce que** l'anticorps monoclonal se lie spécifiquement à la séquence d'acides aminés: TQDVFVGSVEELSAAHTLVMKINATDADEPNTLNSKISYR (SEQ ID NO:1), KINATDADEPNTLNSKISYR (SEQ ID NO:6) ou EELSAAHTLV (SEQ ID NO:7) de la région de sites de reconnaissance d'adhésion non cellulaire (CAR) du domaine EC2 de Dsg2 et inhibe la transition mésenchymateuse épithéliale (EMT).

2. Anticorps monoclonal pour une utilisation selon la revendication 1, où l'anticorps monoclonal est élevé contre la séquence d'acides aminés TQDVFVGSVEELSAAHTLVMKINATDADEPNTLNSKISYR (SEQ ID NO:1), KINATDADEPNTLNSKISYR (SEQ ID NO:6) ou EELSAAHTLV (SEQ ID NO:7)

3. Anticorps polyclonal en tant qu'antagoniste pour une utilisation en tant que médicament pour la prévention ou le traitement d'un cancer, **caractérisé en ce que** l'anticorps polyclonal est élevé contre la séquence d'acides aminés TQDVFVGSVEELSAAHTLVMKINATDADEPNTLNSKISYR (SEQ ID NO:1) ou la SEQ ID NO:5 et inhibe la transition mésenchymateuse épithéliale (EMT).

4. Anticorps monoclonal ou anticorps polyclonal pour une utilisation selon l'une quelconque des revendications 1 à 3, où ledit cancer est un cancer de la prostate, un cancer du sein ou un cancer de la peau.

5. Anticorps monoclonal ou anticorps polyclonal pour une utilisation selon l'une quelconque des revendications 1 à 4, où l'anticorps monoclonal ou l'anticorps polyclonal réduit la fonction favorisant la métastase cellulaire et/ou l'invasion cellulaire de Dsg2.

6. Polypeptide isolé comprenant:
(a) la séquence d'acides aminés TQDVFVGSVEELSAAHTLVMKINATDADEPNTLNSKISYR (SEQ ID NO:1), KINATDADEPNTLNSKISYR (SEQ ID NO:6) ou EELSAAHTLV (SEQ ID NO:7) où ladite séquence d'acides aminés est capable d'inhiber la fonction favorisant la transition mésenchymateuse épithéliale (EMT) de Dsg2; ou
(b) un fragment biologiquement actif de la séquence d'acides aminés TQDVFVGSVEELSAAHTLVMKINATDADEPNTLNSKISYR (SEQ ID NO:1), KINATDADEPNTLNSKISYR (SEQ ID NO:6) ou EELSAAHTLV (SEQ ID NO:7) où ledit fragment est capable d'inhiber la fonction favorisant la transition mésenchymateuse épithéliale (EMT) de Dsg2
pour une utilisation en tant que médicament pour la prévention ou le traitement d'un cancer.

7. Polypeptide isolé comprenant:
(a) la séquence d'acides aminés: SEQ ID NO:9 où ladite séquence d'acides aminés est capable d'inhiber la fonction favorisant la transition mésenchymateuse épithéliale (EMT) de Dsg2
pour une utilisation en tant que médicament pour la prévention ou le traitement d'un cancer.

8. Polypeptide isolé pour une utilisation selon la revendication 6, où le polypeptide comprend la séquence d'acides aminés SEQ ID NO:1.

9. Polypeptide isolé pour une utilisation selon la revendication 6, où le polypeptide comprend la séquence d'acides aminés SEQ ID NO:6.

10. Polypeptide isolé pour une utilisation selon la revendication 6, où le polypeptide comprend la séquence d'acides aminés SEQ ID NO:7.

11. Polypeptide isolé pour une utilisation selon l'une quelconque des revendications 6 à 10, où le polypeptide réduit la fonction favorisant la métastase cellulaire et/ou l'invasion cellulaire de Dsg2.

12. Polypeptide isolé pour une utilisation selon l'une quelconque des revendications 6 à 11, où ledit cancer est un cancer de la prostate, un cancer du sein ou un cancer de la peau.

13. Préparation pharmaceutique comprenant:
(i) un anticorps monoclonal qui se lie spécifiquement à la séquence d'acides aminés: TQDVFVGSVEELSAAHTLVMKINATDADEPNTLNSKISYR (SEQ ID NO:1), KINATDADEPNTLNSKISYR (SEQ ID NO:6) ou EELSAAHTLV (SEQ ID NO:7) de la région de sites de reconnaissance d'adhésion non cellulaire (CAR) du domaine EC2 de Dsg2 et inhibe la transition mésenchymateuse épithéliale (EMT); ou
(ii) un anticorps polyclonal élevé contre la séquence d'acides aminés TQDVFVGSVEELSAAHTLVMKINATDADEPNTLNSKISYR (SEQ ID NO:1) ou la SEQ ID NO:5 qui inhibe la transition mésenchymateuse épithéliale (EMT);
et un excipient pharmaceutiquement acceptable.

14. Utilisation d'un anticorps monoclonal selon l'une quelconque des revendications 1, 2, 4 ou 5 dans la préparation d'un médicament pour la prévention ou le traitement d'un cancer.

15. Utilisation d'un anticorps polyclonal selon l'une quelconque des revendications 3, 4 ou 5 dans la préparation d'un médicament pour la prévention ou le traitement d'un cancer.

16. Utilisation d'un polypeptide isolé selon la revendication 6 dans la préparation d'un médicament pour la prévention ou le traitement d'un cancer.

17. Utilisation d'un polypeptide isolé selon l'une quelconque des revendications 7 à 10 dans la préparation d'un médicament pour la prévention ou le traitement d'un cancer.
